# EUROPEAN PATENT SPECIFICATION

(11) **EP 0 313 094 B1**
(45) Date of publication and mention of the grant of the patent: **01.09.1993**
(21) Application number: 88117611.9
(22) Date of filing: 21.10.1988
(51) Int. Cl.: A61K 31/44

(54) **Anticancer agent and reagent for obtaining revertants**
Anti-Krebs-Mittel und Reagenz zur Gewinnung von Revertanten
Médicament anticancéreux et réactif pour l'obtention des revertants

(30) Priority: 22.10.1987 JP 265379/87
(43) Date of publication of application: 26.04.1989
(73) Proprietor: MEIJI SEIKA KAISHA LTD., Chuo-ku Tokyo 104 (JP)
(72) Inventor: Makabe, Osamu Meiji Seika Kaisha, Ltd., Kohoku-ku Yokohama-shi Kanagawa (JP); Shomura, Takashi Meiji Seika Kaisha, Ltd., Kohoku-ku Yokohama-shi Kanagawa (JP); Nagaoka, Kozo Meiji Seika Kaisha, Ltd., Kohoku-ku Yokohama-shi Kanagawa (JP); Okada, Nobuko National Cancer Center, 1-1, Tsukiji 5-chome Chuo-ku Tokyo (JP); Nishimura, Susumu, Ichihara-shi Chiba (JP)
(74) Representative: Wilhelms, Rolf E., Dr.

(56) References cited:
- FR-A- 2 393 579
- PROC. NATL. ACAD. SCI. USA, vol. 81, August 1984, pages 4771-4775; T. SEKIYA et al.: "Molecular cloning and the total nucleotide sequence of the human c-Ha-ras-1 gene activated in a melanoma from Japanese patient"
- PROC. NATL. ACAD. SCI. USA, vol. 80, September 1983, pages 5602-5606; M. NODA et al.: "Flat revertants isolated from Kirsten sarcoma virus-transformed cells are resistant to the action of specific oncogenes"
- NUCLEIC ACIDS SYMP. RES., Okayama, 20th - 21st February 1988, symposium series no. 19, pages 129-130, IRL Press Ltd, Oxford, GB; N. SHINDO-OKADA et al.: "The antibiotic, L-beta-(5-hydroxy-2-pyridyl)-alanine specifically inhibits growth of the NIH3T3 cells transformed by activated human c-Ha-ras and induces formation of flat revertant cells"

## Description

### FIELD OF THE INVENTION

The present invention relates to an anticancer agent comprising L-β-(5-hydroxy-2-pyridyl)alanine as an active ingredient and a reagent for obtaining reverse mutant cells comprising the same.

### BACKGROUND OF THE INVENTION

Known oncogenes found in cells human cancer patients include ras, raf, hst, myc, ret and lca. On the other hand, herbimycin (cf. Y. Uehara et al., Jpn. J. Cancer Res. (Gann), 76, 672 (1905)) and oxanosine (cf. Y. Uehara et al., Biochem. J., 232, 825 (1985)) are known as inhibitors against src which is an oncogene found in animals but has never been isolated from human cancer patients so far. However, there has been found no substance capable of selectively inhibiting the growth of cancer cells which are transformed with an oncogene such as ras or raf isolated from a human cancer patient.

M. Noda et al. reported a process for obtaining revertant cells (converted cells), which are morphologically similar to normal ones and produce an oncogene product without showing any transforming activity, with the use of a specific chemical, for example, ouabain (cf. Proc. Natl. Acad. Sci. USA, 80, 5602 (1983)). However, there has been reported no method whereby a revertant can be readily obtained with the use of a single chemical.

L-β-(5-hydroxy-2-pyridyl)alanine is known as an antibiotic SF-1346 which is isolated from a culture medium of Streptomyces chibaensis SF-1346 strain which has been deposited at Fermentation Research Institute under the deposition number of FERM BP-1536 in accordance with Budapest treaty. The compound is represented by the following formula: 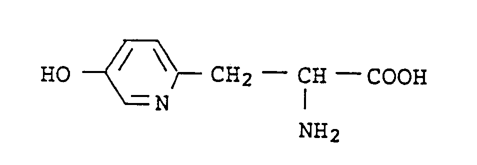
This compound, which exerts an antibacterial and antifungal activity, is described in JP-B-54-39479 (the term "JP-B" used herein means an "examined published Japanese patent application) in detail.

### SUMMARY OF THE INVENTION

We have found that SF-1346 selectively inhibits the growth of NIH3T3 cells which are transformed, i.e., cancerized with activated melanoma c-H-ras (T. Sekiya et al., Proc. Natl. Acad. Sci. USA, 81, 4771 (1984)) at a concentration of 500 µg/mℓ but does not inhibit the growth of normal NIH3T3 cells at the same concentration, as will be shown by the following Examples. We have further found that the cells surviving after the above treatment are reverse mutant cells, which suggests that the antibiotic SF-1346 is useful not only as an anticancer agent but also as a reagent for obtaining revertants. The same result as the one described above is achieved by carrying out a similar test with the use of another oncogene raf. Although these revertants produce oncogene products, they show scarcely any oncogenesis when administered to nude mice. They have a flat shape and are closely similar to normal cells from a morphological viewpoint. In addition, they can be subcultured. Thus, the present invention was completed.

Accordingly, an object of the invention is to provide an anticancer agent suitable for the treatment of cancers in mammals which comprises L-β-(5-hydroxy-2-pyridyl)alanine as an active ingredient. Another object of the invention is to provide a reagent for obtaining revertants which comprises L-β-(5-hydroxy-2-pyridyl)alanine.

### BRIEF DESCRIPTION OF THE DRAWINGS

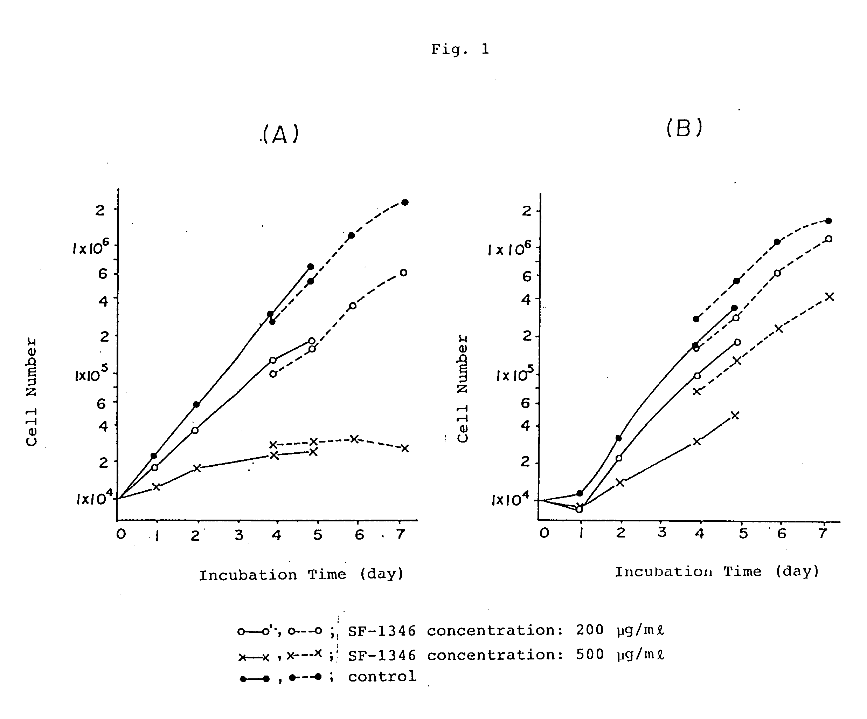

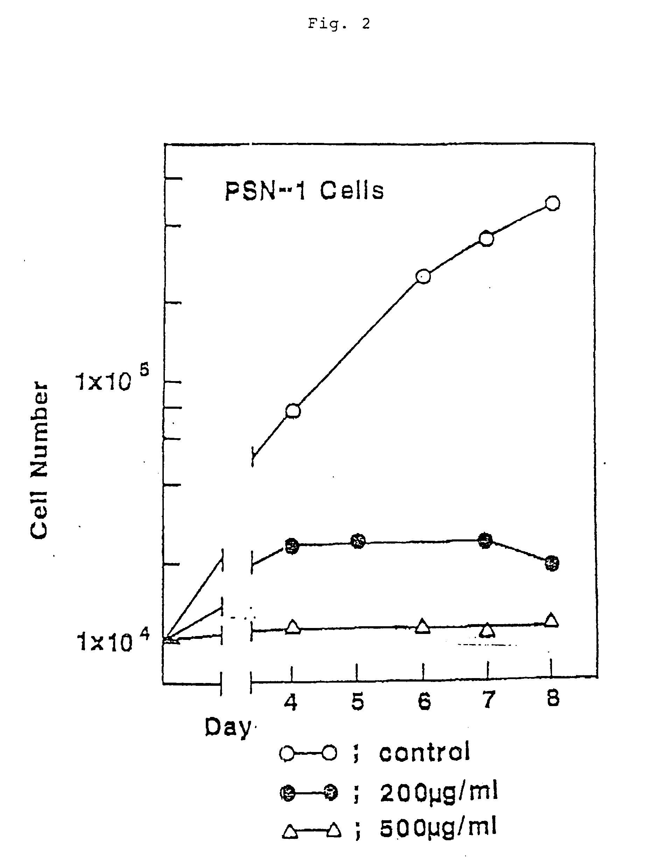

Fig. 1 and Fig. 2 are each a graph which shows the growth-inhibition effect of the compound of the present invention. In Fig. 1, (A) illustrates the result regarding transformed NIH3T3 cells, while (B) illustrates the result regarding normal NIH3T3 cells. Fig. 2 illustrates the result regarding PSN-1 cells.

### DETAILED DESCRIPTION OF THE INVENTION

The antibiotic SF-1346, i.e., L-β-(5-hydroxy-2-pyridyl)alanine to be used in the present invention may be prepared according to the method disclosed in JP-B-54-39479.

When the compound of the present invention is to be used as an anticancer agent, it may be parenterally or orally administered. The parenteral administration can be effected by intravenous, subcutaneous or intramuscular injection. The compound of the present invention may be formulated into an injectable solution or suspension together with a physiological saline or a pharmaceutically acceptable dispersant such as gum arabic and Tween^{R} 80. In the case of oral administration, it may be mixed with, for example, pharmaceutically acceptable carriers and filled into a gelatin capsule, if desired, Alternately, it may be formulated into a composition together with medicine(s), starch lubricant(s) and other pharmaceutically acceptable carriers in such a manner as to give a content of the active ingredient of 125 to 250 mg and then formulated into a tablet. The compound of the present invention should be administered to mammals including man in a dose of 10 to 500 mg/kg body weight, preferably 100 to 500 mg/kg body weight, per day. The dosage may vary depending on the age, body weight, conditions and therapeutic reactions of the patient.

The acute toxicity of the compound of the present invention is examined by intravenously injecting 100 mg portions of the same into five ICR mice aged five weeks once. As a result, every animal survived two weeks or longer thereafter.

The process for obtaining revertant cells according to the present invention comprises transfecting an oncogene into cultured cells, incubating the transformed cells in the presence of SF-1346 and recovering the surviving cells.

Examples of oncogenes used for transformation include c-H-ras, c-Ki-ras, N-ras, ret, hst, raf and src. Suitable cultured cells to be transformed include NIH3T3, 3Y1 and PSN-1. Incubation of the transformed cells may be carried out in the manner suitable for the growth of the cultured cell used. The revertant cells obtained can be recovered by a known technique in the art.

To further illustrate the present invention, the following Examples will be given.

### EXAMPLE 1

### Specific inhibition of the growth of transformed NIH3T3 cells by SF-1346

NIH3T3 cells transformed by an activated melanoma c-H-ras oncogene and normal NIH3T3 cells were incubated at 37°C each in a Dulbecco-modified Eagle's medium containing 5% (v/v) of bovine serum.

Each medium was adjusted to have an initial cell density of 1 × 10⁴ cell/mℓ and contain SF-1346 at a concentration of 200 µg/mℓ or 500 µg/mℓ. The cells therein were counted with the lapse of time from the first day to the fifth day and from the fourth day to the seventh day of the incubation. Fig. 1 shows the results.

Fig. 1 obviously indicates that the SF-1346 inhibit the growth of the transformed NIH3T3 cells at the concentration of 500 µg/mℓ but does not inhibit that of the normal NIH3T3 cells at the same concentration.

### EXAMPLE 2

SF-1346 were added to the culture medium of NIH3T3 cells transformed with activated c-Ki-ras, N-ras, ret, hst and raf oncogenes in the same manner as in Example 1 so as to give a final concentration of 500 µg/mℓ. As a result, SF-1346 inhibited the growth of the transformed NIH3T3 cells but did not inhibit that of the normal cells at the same concentration, similar to the case of Example 1. In addition, SF-1346 completely inhibited the growth of SRY1 cells which had been obtained by cancerizing rat fibroblast 3Y1 cells through transfection with an src oncogene.

### EXAMPLE 3

### Aquirement of revertants from transformed NIH3T3 cells with the use of SF-1346

Cell incubation was carried out under the same conditions as those described in Example 1 except that SF-1346 was added to the culture medium so as to give a final concentration of 500 µg/mℓ. On the sixth day of the incubation, no morphological change was observed as to the normal cells, while observation with an inverted microscope at 100 × magnification revealed that the transformed cells partially died and the selectively surviving cells were morphologically similar to the normal NIH3T3 cells. Namely, these surviving revertants formed flat foci.

Then, the revertants were isolated by selecting cell clusters forming flat foci by penicillin capping. After repeating this procedure several times, the desired revertants were isolated.

Separately, the above process was repeated except using NIH3T3 cells transformed with an activated c-Ki-ras, N-ras or raf oncogene. As a result, similar revertants as described above could be obtained.

### EXAMPLE 4

### Inhibition of the growth of PSN-1 cells derived from human pancreatic cancer and aquirement of revertants

PSN-1 cells were incubated at 37°C in RPMI 1640 media each containing 10% (v/v) fetal calf serum and SF-1346 at a concentration of 500 µg/mℓ or 200 µg/mℓ. An initial cell density was adjusted to 1 × 10⁴ cells/mℓ. The cells were counted with the lapse of time from the fourth day to the eighth day after the initiation of the incubation. Fig. 2 shows the results.

Fig. 2 obviously indicates that SF-1346 completely killed the PSN-1 cells at the concentration of 500 µg/mℓ. At the concentration of 200 µg/mℓ, SF-1346 killed 15% of the cells and converted the residual 85% of the same into flat revertants, thus completely inhibiting the growth of the cancer cells.

### TEST EXAMPLE 1

### Doubling time

The revertants isolated in Example 3, the transformed NIH3T3 cells and normal NIH3T3 cells were cultivated at 37°C in a Dulbecco-modified Eagle's medium containing 5% (v/v) of bovine serum. Doubling time of the transformed NIH3T3 cells and normal NIH3T3 cells were determined 48 hours after the initiation of the incubation from the growth curve. In the case of the revertants, doubling time was determined by microscopic observation. The results are shown in Table 1.

### TEST EXAMPLE 2

### Expression of a p21 oncogene in revertants

2 × 10⁶ cells of the revertants isolated in Example 3, the transformed NIH3T3 cells and normal NIH3T3 cells were collected in order to examine the expression of a p21 oncogene in these cells. Cells were suspended in 1 mℓ of a buffer solution (20 mM tris-hydrochloride (pH 7.5), 5 mM MgCl₂, 1% NP-40, 0.5% sodium deoxycholate, 5 µg/mℓ protease inhibitor) and then ground with a Potter homogenizer. After centrifuging at 3,600 rpm for 60 minutes, 10 µg of protein was obtained from the supernatant. The thus-obtained protein was fractionated by 12.5% polyacrylamide gel electrophoresis.

Subsequently, the protein in the gel was transferred into a nitrocellulose membrane and reacted with Y13-259 or Ncc ras 004, each a monoclonal antibody for p21. The reaction mixture was further reacted with rat Ig and ¹²⁵I-labeled protein A and then subjected to radioautography. The results are shown in Table 1 below.

From the results shown in Table 1, it was found that the p21 was produced in the revertants as well as in the transformed NIH3T3 cells.

### TEST EXAMPLE 3

### GTP-binding activity of p21 produced in revertants

The GTP-binding activity of the p21 produced in the revertants was examined by the following method. Namely, the p21 produced was transferred from a 125% polyacrylamide gel to a nitrocellulose filter in the same manner as in Example 3, and the nitrocellulose membrane was immersed in a buffer (50 mM tris-hydrochloride (pH 7.5), 5 mM MgCl₂ 0.1% NP-40, 1 mg/mℓ calf serum albumin) containing 150 µCi of [α-³²P]-GTP and allowed to react therein at 37°C for one hour, followed by subjecting to radioautography. The results are shown in Table 1.

It is apparent from the results shown in Table 1 that the revertants exerts a similar GTP-binding activity to that of the transformed NIH3T3 cells.

### TEST EXAMPLE 4

### Oncogenic effect of revertants on nude mouse

The revertants isolated in Example 3 (3 × 10⁵ cells), the transformed NIH3T3 cells (3 × 10⁴ cells and 3 × 10⁵ cells) and normal NIH3T3 cells (3 × 10⁵ cells were transplanted each to both shoulders of a six-week-old BALB/C nude mouse and after 40 days of feeding, oncogenesis on the shoulder of each mouse was examined. The results are shown in Table 1.

The results shown in Table 1 obviously indicate that oncogenic effect of the transformed cells was observed either at the dose of 3 × 10⁵ cells/shoulder or that of 3 × 10⁴ cells/shoulder, while that of the revertant cells was scarcely observed even at the concentration of 3 × 10⁵ cells/shoulder.

**Table 1**

| Properties of revertants | | | |
|---|---|---|---|
| | Transformed NIH3T3 cell | Normal NIH3T3 cell | Revertant NIH3T3 cell |
| Doubling time (hr) (Test Example 1) | 19.2 | 19.2 | 24.0 |
| Expression of P21 gene (Test Example 2) | + | - | + |
| GTP-binding activity (Test Example 3) | + | - | + |
| Oncogenic effect (Test Example 4) | + (3 × 10⁴>) | - | ± (3 × 10⁵<) |

It is expected that the compound SF-1346, i.e., L-β-(5-hydroxy-2-pyridyl)alanine is useful as an excellent anticancer agent and as an effective reagent for obtaining revertants.

While the invention has been described in detail and with reference to specific embodiments thereof, it will be apparent to one skilled in the art that various changes and modifications can be made.

## Claims

1. A process for obtaining revertant cells comprising transforming cultured cells with an oncogene, incubating the transformed cells in the presence of L-β-(5-hydroxy-2-pyridyl)alanine, and recovering the surviving cells.

2. The use of L-β-(5-hydroxy-2-pyridyl)alanine for the manufacture of an anticancer agent for the treatment of cancers.

3. The use of L-β-(5-hydroxy-2-pyridyl)alanine for obtaining revertants.

## Patentansprüche

1. Verfahren zum Erhalt revertanter Zellen wobei kultivierte Zellen mit einem Onkogen transformiert, die transformierten Zellen in Gegenwart von L-β-(5-Hydroxy-2-pyridyl)alanin inkubiert und die überlebenden Zellen gewonnen werden.

2. Verwendung von L-β-(5-Hydroxy-2-pyridyl)alanin zur Herstellung eines Antikrebsmittels für die Behandlung von Krebsarten.

3. Verwendung von L-β-(5-Hydroxy-2-pyridyl)alanin zum Erhalt von Revertanten.

## Revendications

1. Procédé pour l'obtention de cellules révertantes comprenant la transformation de cellules cultivées avec un oncogène, l'incubation des cellules transformées en présence de L-β-(5-hydroxy-2-pyridyl)alanine, et la récupération des cellules survivantes.

2. Utilisation de la L-β-(5-hydroxy-2-pyridyl)alanine pour la fabrication d'un agent anti-cancéreux pour le traitement des cancers.

3. Utilisation de la L-β-(5-hydroxy-2-pyridyl)alanine pour l'obtention de révertants.
